# EUROPEAN PATENT SPECIFICATION

(11) **EP 2 653 171 B1**
(45) Date of publication and mention of the grant of the patent: **25.03.2015**
(21) Application number: 13163520.3
(22) Date of filing: 12.04.2013
(51) Int. Cl.: A61L 9/04, A61L 9/12, A45D 34/02

(54) **Fragrance bottle**
Duftstoffflasche
Flacon de parfum

(30) Priority: 18.04.2012 CN 201210115810
(43) Date of publication of application: 23.10.2013
(73) Proprietor: Zhongshan Fuji Chemical Co., Ltd., 528436 Zhongshan Guangdong (CN)
(72) Inventor: FU, Feifei, 528436 Zhongshan Guangdong (CN)
(74) Representative: Hryszkiewicz, Danuta

(56) References cited:
- WO-A1-2011/120073
- WO-A1-2012/029504
- US-A1- 2011 139 890
- US-A1- 2012 061 486

## Description

This invention relates to a bottle, and more particularly to a fragrance bottle according to the preamble of claim 1.

People like to use aroma oils to keep a fresh smell in their houses, and aroma twig diffusers are very popular in the market. A typical bottle for receiving aroma twig diffusers includes a bottle body and a bottle cap including holes; twigs are introduced through the holes of the cap, and directly inserted into the bottle body and immersed in the volatile matter. This structure is disadvantageous in that when the bottle is knocked down, the volatile matter in the bottle body quickly flows out of the bottle body, thereby resulting in waste. In the quest for a solution, WO 2011/120073 A1 discloses a fragrance diffuser which comprises a container, a spill guard, a decorative wooden collar, and at least one rattan reed. The opening of the container is plugged by the spill guard, and the opening of the container and the spill guard are both covered by the decorative wooden collar. The rattan reed is inserted into the container through the holes on the spill guard and the decorative wooden collar. However, when the fragrance diffuser is tipped over, the decorative wooden collar falls off and twists the rattan reed, and the fragrant liquid in the container flows out from the container through the holes on the spill guard.

In view of the above-described problems, it is one objective of the invention to provide a fragrance bottle that has a simple structure, and can effectively prevent the inner fluids from leaking out of the bottle body when the bottle is knocked down.

Technical scheme of the invention is as follows:

A fragrance bottle comprises: a bottle body, the bottle body comprising a first opening and an inner cavity; a bottle cap, the bottle cap comprising a through hole; a bottle stopper, the bottle stopper comprising a plurality of holes. The bottle cap is disposed on the bottle body. The through hole is arranged on a top of the bottle cap. The bottle stopper is inserted into the first opening for sealing. The holes are in communication with the inner cavity of the bottle body.

The bottle stopper comprises: an upper end, and a connecting part. The connecting part is integrated with the upper end. The connecting part is plugged into the first opening of the bottle body.

The connecting part is in a shape of a conical cylinder. The connecting part comprises an outer wall; and an annular convex is disposed on the outer wall of the connecting part.

The bottle cap is in a shape of a cylinder. The bottle cap comprises: the top, and a bottom. A second opening is arranged on the bottom of the bottle cap. The second opening is sleeved on the bottle body. A diameter of the second opening is larger than a diameter of the top of the bottle cap.

The first opening of the bottle body is in a shape of a cylinder; and an external thread is arranged on an outer wall of the first opening.

An annular groove is arranged on a middle part of the bottle body. The second opening of the bottle cap comprises a convex clasp; the convex clasp is arranged on an inner wall of an end of the second opening; and the convex clasp matches with the annular groove.

A first recess is disposed on the top of the bottle cap; and the through hole is arranged on a bottom of the first recess.

The number of the holes arranged on the bottle stopper is seven; a diameter of each hole is 3.3 mm. One of the holes is arranged on a center of the upper end of the bottle stopper; and the other six holes are evenly distributed surrounding the center.

A twig is inserted into the through hole of the bottle cap; and a lower end of the twig is extended into the bottle body after passing through the holes arranged on the bottle stopper.

A second recess having a depth of 0.5 mm is arranged on the upper end of the bottle stopper. The connecting part comprises a cylinder cavity; and the cylinder cavity is in communication with the holes of the bottle stopper and the bottle body.

The bottle stopper is inserted into the first opening of the bottle body for sealing, and a plurality of holes are arranged on the bottle stopper. The size of the hole is comparable to the diameter of the twig, thus, when the twigs are inserted into the holes of the bottle stopper, it assures that in condition that the bottle is knocked down, the inner fluid is prevented from leaking out of the bottle via the holes of the bottle stopper or other parts. The twig is extended to the volatile fluid after passing the through hole of the bottle cap and the holes of the bottle stopper. The bottle stopper comprises: the upper end, and the connecting part. The connecting part and the upper end are connected as a whole. The connecting part can be inserted into the first opening of the bottle body. The connecting part is a conical cylinder, which is very convenient to plug the bottle stopper into the first opening of the bottle body. A plurality of holes are arranged on the upper end of the bottle stopper. An annular convex is arranged on the outer wall of the connecting part. A diameter of the annular convex is a bit larger than a diameter of the inner wall of the first opening to assure a tight match between the connecting part and the first opening. The holes are evenly arranged on the upper end of the bottle stopper. A total of seven of the holes are arranged on the bottle stopper; the diameter of each hole is 3.3 mm. One of the holes is arranged on the center of the upper end of the bottle stopper; and the other six holes are evenly distributed surrounding the center. The second recess having the depth of 0.5 mm is arranged on the upper end of the bottle stopper. The cylinder cavity is in communication with the holes of the bottle stopper and the bottle body.

The fragrance bottle of the invention has a simple structure, and effectively prevents the volatile fluid from quickly leaking out of the fragrance bottle.

The invention is described hereinbelow with reference to the accompanying drawings, in which:
FIG. 1 is an assembly diagram of a fragrance bottle of the invention;
FIG. 2 is an exploded view of a fragrance bottle of the invention;
FIG. 3 is a stereograph view of a bottle stopper; and

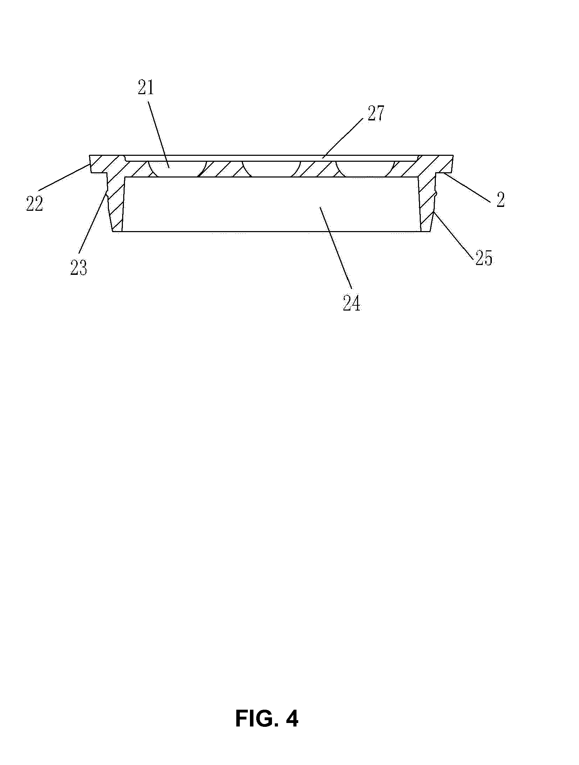

FIG. 4 is a cross-sectional view of a bottle stopper.

For further illustrating the invention, experiments detailing a fragrance bottle are described below. It should be noted that the following examples are intended to describe and not to limit the invention.

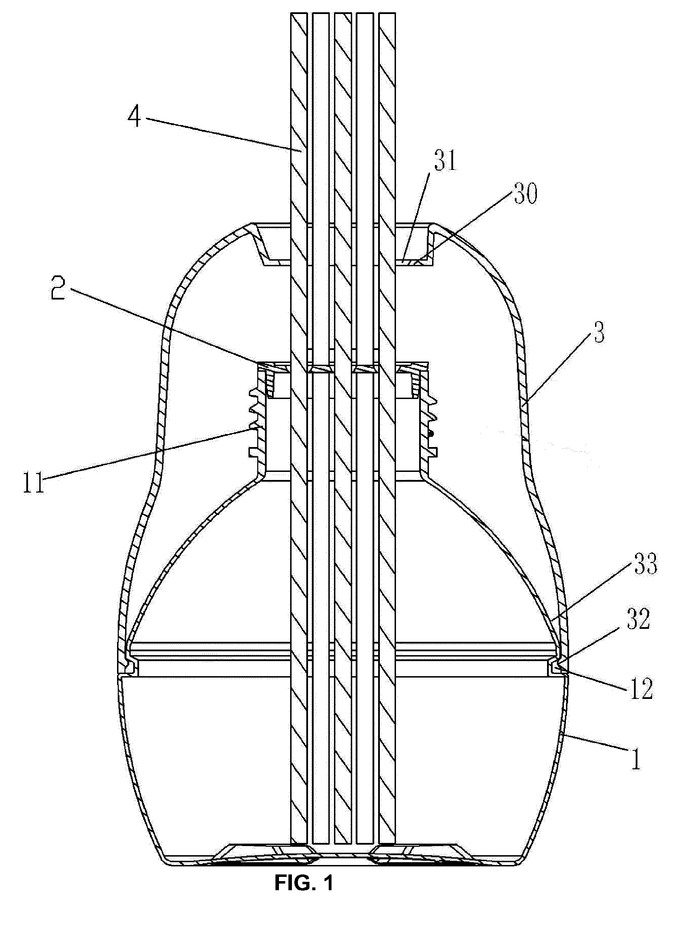

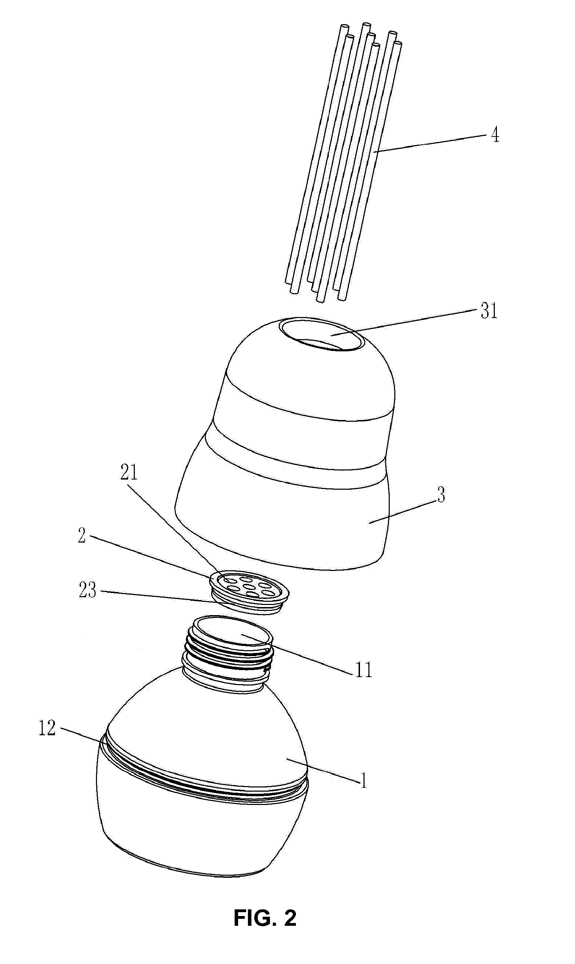

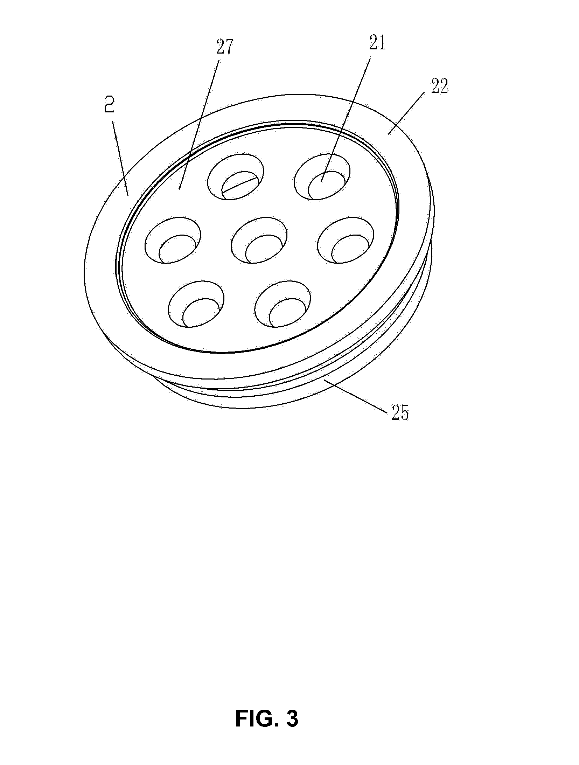

As shown in FIG. 1, a fragrance bottle comprises a bottle body 1 for filling an aroma fluid. The bottle body 1 comprises: an annular groove 12, an inner cavity, and a first opening 11. The annular groove 12 is arranged on a middle part of the bottle body 1. A bottle cap 3 is disposed on the first opening 11. The bottle cap 3 comprises: a top, and a bottom. A through hole 31 is arranged on the top, and a second opening 33 is arranged on the bottom, and the second opening 33 is sleeved on the bottle body. A diameter of the second opening 33 is larger than a diameter of the top of the bottle cap 3. A convex clasp 32 is arranged on an inner wall of an end of the second opening 33, and the convex clasp 32 matches the annular groove 12 of the bottle body 1. A bottle stopper 2 that tightly fits with the first opening 11 is inserted in the first opening 11. As shown in FIG. 2 and 3, a plurality of holes 21 are arranged on the bottle stopper 2, the holes 21 are in communication with the inner cavity of the bottle body 1. A diameter of the hole 21 is comparable with a diameter of a twig 4, thus, when the twigs are inserted to the holes of the bottle stopper, it assures that in condition that the bottle is knocked down, the volatile fluid inside is prevented from leaking out of the bottle body 1 from the holes 21 of the bottle stopper or other parts. The twigs 4 are inserted into the bottle body 1 and immersed into the volatile fluid after passing the through hole 31 of the bottle cap 3 and the holes 21 arranged on the bottle stopper 2. The volatile fluid spreads along the twigs 4 from a lower end to an upper end, and spreads into the air via the holes 21 and the through hole 31.

A first recess 30 is arranged on the top of the bottle cap 3, the through hole is arranged on a bottom of the first recess 30. Thus, when the twigs are inserted into the bottle body, the through hole is functions in supporting 4 in case of falling or bending of the twigs 4.

The bottle stopper 2 comprises: an upper end 22, and a connecting part 25. The connecting part 25 is integrated with the upper end 22; and the connecting part 25 is plugged into the first opening 11 of the bottle body 1. A plurality of the holes are arranged on the upper end of the bottle stopper 2. The connecting part 25 comprises an outer wall; and an annular convex is disposed on the outer wall of the connecting part 25. A diameter of the annular convex 23 is a bit larger than the diameter of the first opening 11 of the bottle body 1, which assures a good sealing property when the connecting part is inserted in the first opening 11. The connecting part 25 is in a shape of a conical cylinder, which is conducive to the sealing between the bottle stopper 2 and the first opening 11. The number of the holes 21 arranged on the bottle stopper 2 is seven. A diameter of each hole 21 is 3.3 mm. One of the holes 21 is arranged on a center of the upper end 22 of the bottle stopper 2; and the other six holes 21 are evenly distributed surrounding the center.

A second recess 27 having a depth of 0.5 mm is arranged on the upper end 22 of the bottle stopper 2. The connecting part 25 comprises a cylinder cavity 24; and the cylinder cavity 24 is in communication with the holes 21 of the bottle stopper 2 and the bottle body 1. In use, the twigs 4 are inserted into the through hole 31 of the bottle cap 3, and then introduced through the holes 21 of the bottle stopper 2, respectively. The connecting part 25 of the bottle stopper 2 is plugged into the first opening 11. The lower parts of the twigs are immersed into the volatile matters. Finally, the second opening 33 of the bottle cap 3 is sleeved on the bottle body 1, and the convex clasp 32 of the bottle cap 3 is received by the annular groove 12 on the bottle body 1.

The first opening 11 of the bottle body 1 is in a shape of a cylinder; and an external thread is arranged on an outer wall of the first opening 11. When a fragrance bottle is produced, a second bottle cap (not shown in FIGS.) is threaded on the first opening for sealing the bottle body. To use the product, at first the second bottle cap is removed from the first opening, then, the twigs are introduced through the through hole 31 of the bottle cap 3 and the holes 21 of the bottle stopper 31 until the lower parts of the twigs are immersed into the inner fluid.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the scope of the invention.

## Claims

1. A fragrance bottle, comprising:
a) a bottle body (1) comprising a first opening (11) and an inner cavity;
b) a bottle cap (3) comprising a through hole (31), a second opening, a top, and a bottom; the bottle cap (3) being disposed on the bottle body (1), the through hole (31) being arranged on the top of the bottle cap (3), and the second opening (33) being arranged on the bottom of the bottle cap (3); and
c) a bottle stopper (2) comprising a plurality of holes (21), the bottle stopper (2) being inserted into the first opening (11) for sealing, and the holes (21) being communicated with the inner cavity of the bottle body (1); **characterized in that**
an annular groove (12) is arranged on a middle part of the bottle body (1);
the second opening (33) of the bottle cap (3) comprises a convex clasp (32);
the convex clasp (32) is arranged on an inner wall of an end of the second opening (33); and
the convex clasp (32) is matched with the annular groove (12).

2. The bottle of claim 1, **characterized in that**
the bottle stopper (2) further comprises: an upper end (22), and a connecting part (25);
the connecting part (25) is integrated with the upper end (22); and the connecting part (25) is plugged into the first opening (11) of the bottle body (1).

3. The bottle of claim 2, **characterized in that**
the connecting part (25) is in a shape of a conical cylinder;
the connecting part (25) comprises an outer wall; and
an annular convex is disposed on the outer wall of the connecting part (25).

4. The bottle of claim 1, **characterized in that**
the bottle cap (3) is in a shape of a cylinder;
the second opening (33) is sleeved on the bottle body (1); and
a diameter of the second opening (33) is larger than a diameter of the top of the bottle cap (3).

5. The bottle of claim 1, **characterized in that**
the first opening (11) of the bottle body (1) is in a shape of a cylinder; and
an external thread is arranged on an outer wall of the first opening (11).

6. The bottle of claim 1, **characterized in that** a first recess (30) is disposed on the top of the bottle cap (3);
and the through hole (31) is arranged on a bottom of the first recess (30).

7. The bottle of claim 1, **characterized in that**
the number of the holes (21) arranged on the bottle stopper (2) is seven;
a diameter of each hole (21) is 3.3 mm;
one of the holes (21) is arranged on a center of the upper end (22) of the bottle stopper (2); and
the other six holes (21) are evenly distributed surrounding the center.

8. The bottle of claim 1, **characterized in that** a twig (4) is inserted into the through hole (31) of the bottle cap (3); and a lower end of the twig (4) is extended into the bottle body after passing through the holes (21) arranged on the bottle stopper (2).

9. The bottle of claim 2, **characterized in that** a second recess (27) having a depth of 0.5 mm is arranged on the upper end (22) of the bottle stopper (2); the connecting part (25) comprises a cylinder cavity (24); and the cylinder cavity (24) is in communication with the holes (21) of the bottle stopper (2) and the bottle body (1).

## Patentansprüche

1. Duftstoffflasche umfassend:
a) einen Flaschenkörper (1), der eine erste Öffnung (11) und einen Innenhohlraum umfasst;
b) eine Flaschenkappe (3), die ein Durchgangsloch (31), eine zweite Öffnung, ein Kopfende und einen Boden umfasst; wobei die Flaschenkappe (3) sich auf dem Flaschenkörper (1) befindet, das Durchgangsloch (31) oben an der Flaschenkappe (3) angeordnet ist und die zweite Öffnung (33) unten an der Flaschenkappe (3) angeordnet ist; und
c) einen Flaschenstöpsel (2), der mehrere Löcher (21) umfasst, wobei der Flaschenstöpsel (2) in die erste Öffnung (11) zum dicht Verschließen eingeführt ist und die Löcher (21) mit dem Innenhohlraum des Flaschenkörpers (1) in Kommunikation stehen;
**dadurch gekennzeichnet, dass**
eine ringförmige Rille (12) am Mittelteil des Flaschenköpers (1) angeordnet ist;
die zweite Öffnung (33) der Flaschenkappe (3) eine konvexe Klammer (32) umfasst;
die konvexe Klammer (32) an einer Innenwand eines Endes der zweiten Öffnung (33) angeordnet ist; und
die konvexe Klammer (32) mit der ringförmigen Rille (12) zusammenpasst.

2. Flasche nach Anspruch 1, **dadurch gekennzeichnet, dass**
der Flaschenstöpsel (2) ferner Folgendes umfasst: ein oberes Ende (22) und ein Verbindungsteil (25);
wobei das Verbindungsteil (25) mit dem oberen Ende (22) integriert ist; und
das Verbindungsteil (25) in die erste Öffnung (11) des Flaschenkörpers (1) eingesteckt ist.

3. Flasche nach Anspruch 2, **dadurch gekennzeichnet, dass**
das Verbindungsteil (25) in der Form eines konischen Zylinders vorliegt;
das Verbindungsteil (25) eine Außenwand umfasst; und
ein ringförmiges Konvexteil sich an der Außenwand des Verbindungsteils (25) befindet.

4. Flasche nach Anspruch 1, **dadurch gekennzeichnet, dass**
die Flaschenkappe (3) in der Form eines Zylinders vorliegt;
die zweite Öffnung (33) auf den Flaschenkörper (1) hülsenförmig aufgebracht ist; und
ein Durchmesser der zweiten Öffnung (33) größer ist als ein Durchmesser des Kopfendes der Flaschenkappe (3).

5. Flasche nach Anspruch 1, **dadurch gekennzeichnet, dass**
die erste Öffnung (11) des Flaschenkörpers (1) in Form eines Zylinders vorliegt; und
ein Außengewinde an einer Außenwand der ersten Öffnung (11) angeordnet ist.

6. Flasche nach Anspruch 1, **dadurch gekennzeichnet, dass** eine erste Vertiefung (30) sich auf dem Kopfende der Flaschenkappe (3) befindet; und das Durchgangsloch (31) an einem Boden der ersten Vertiefung (30) angeordnet ist.

7. Flasche nach Anspruch 1, **dadurch gekennzeichnet, dass**
die Anzahl von Löchern (21), die an dem Flaschenstöpsel (2) angeordnet sind, 7 beträgt;
ein Durchmesser jedes Lochs (21) 3,3 mm beträgt;
eines der Löcher (21) auf einer Mitte des oberen Endes (22) des Flaschenstöpsels (2) angeordnet ist; und
die anderen sechs Löcher (21) gleichmäßig die Mitte umgebend verteilt sind.

8. Flasche nach Anspruch 1, **dadurch gekennzeichnet, dass** ein Zweig (4) in das Durchgangsloch (31) der Flaschenkappe (3) eingeschoben ist; und ein unteres Ende des Zweigs (4) sich in den Flaschenkörper erstreckt, nachdem er durch die Löcher (21), die am Flaschenstöpsel (2) angeordnet sind, hindurchgegangen ist.

9. Flasche nach Anspruch 2, **dadurch gekennzeichnet, dass** eine zweite Vertiefung (27), die eine Tiefe von 0,5 mm aufweist, an dem oberen Ende (22) des Flaschenstöpsels (2) angeordnet ist; das Verbindungsteil (25) einem Zylinderhohlraum (24) umfasst; und der Zylinderhohlraum (24) in Kommunikation mit den Löchern (21) des Flaschenstöpsels (2) und dem Flaschenkörper (1) steht.

## Revendications

1. Flacon de parfum, comprenant :
a) un corps de flacon (1) comprenant une première ouverture (11) et une cavité interne ;
b) un capuchon de flacon (3) comprenant un trou traversant (31), une seconde ouverture, un dessus et un dessous ; le capuchon de flacon (3) étant disposé sur le corps de flacon (1), le trou traversant (31) étant pratiqué sur le dessus du capuchon de flacon (3), et la seconde ouverture (33) étant pratiquée sur le dessous du capuchon de flacon (3) ; et
c) un arrêt de flacon (2) comprenant une pluralité de trous (21), l'arrêt de flacon (2) étant inséré dans la première ouverture (11) pour l'étanchéité et les trous (21) étant en communication avec la cavité interne du corps de flacon (1) ;
**caractérisé en ce**
**qu**'une gorge annulaire (12) est disposée dans une partie médiane du corps de flacon (1) ;
la seconde ouverture (33) du capuchon de flacon (3) comprend un fermoir convexe (32) ;
le fermoir convexe (32) est disposé sur une paroi interne de la seconde ouverture (33) ; et
le fermoir convexe (32) est adapté à la gorge annulaire (12).

2. Flacon selon la revendication 1, **caractérisé en ce que**
l'arrêt de flacon (2) comprend en outre : une extrémité supérieure (22), et une pièce de connexion (25) ;
la pièce de connexion (25) est intégrée dans l'extrémité supérieure (22) ; et
la pièce de connexion (25) est fichée dans la première ouverture (11) du corps de flacon (1).

3. Flacon selon la revendication 2, **caractérisé en ce que**
la pièce de connexion (25) a une forme de cylindre conique ;
la pièce de connexion (25) comprend une paroi externe ; et
un convexe annulaire est disposé sur la paroi externe de la pièce de connexion (25).

4. Flacon selon la revendication 1, **caractérisé en ce que**
le capuchon de flacon (3) a une forme de cylindre ;
la seconde ouverture (33) est emmanchée sur le corps de flacon (1) ; et
un diamètre de la seconde ouverture (33) est supérieur à un diamètre du dessus du capuchon du flacon (3).

5. Flacon selon la revendication 1, **caractérisé en ce que**
la première ouverture (11) du corps de flacon (1) a une forme de cylindre ; et
un filetage extérieur est disposé sur une paroi externe de la première ouverture (11).

6. Flacon selon la revendication 1, **caractérisé en ce qu**'un premier retrait (30) est disposé sur le dessus du capuchon du flacon (3) ; et que le trou traversant (31) est pratiqué sur un dessous du premier retrait (30).

7. Flacon selon la revendication 1, **caractérisé en ce que**
le nombre de trous (21) pratiqués sur l'arrêt de flacon (2) est de sept ;
le diamètre de chacun des trous (21) est de 3,3 mm ;
un des trous (21) est pratiqué à un centre de l'extrémité supérieure (22) de l'arrêt de flacon (2) ; et
les six autres trous (21) sont répartis régulièrement autour du centre.

8. Flacon selon la revendication 1, **caractérisé en ce qu**'un rameau (4) est inséré dans le trou traversant (31) du capuchon du flacon (3) ; et une extrémité inférieure du rameau (4) s'étend dans le corps de flacon après avoir traversé les trous (21) pratiqués sur l'arrêt de flacon (2).

9. Flacon selon la revendication 2, **caractérisé en ce qu**'un second retrait (27) ayant une profondeur de 0,5 mm est disposé à l'extrémité supérieure (22) de l'arrêt de flacon (2) ; la pièce de connexion (25) comprend une cavité de cylindre (24) ; et la cavité de cylindre (24) est en communication avec les trous (21) de l'arrêt de flacon (2) et du corps de flacon (1).
